Europäisches Patentamt

**European Patent Office** (11) Publication number: **0 024 206**

**Office européen des brevets** **A2**

# (12) EUROPEAN PATENT APPLICATION

(21) Application number: **80302813.3**

(22) Date of filing: **14.08.80**

(51) Int. Cl.³: **C 12 P 21/00**
**A 61 K 35/74, A 61 K 37/02**

(30) Priority: **16.08.79 JP 104713/79**

(43) Date of publication of application:
**25.02.81 Bulletin 81/8**

(84) Designated Contracting States:
**CH DE FR GB IT LI SE**

(71) Applicant: **SUMITOMO CHEMICAL COMPANY LIMITED**
**15 Kitahama 5-chome Higashi-ku**
**Osaka-shi Osaka-fu(JP)**

(72) Inventor: **Yoshida, Noboru**
**5-3-618 Higashiawajicho 1-chome**
**Higashiyodogawa-ku Osaka 533(JP)**

(72) Inventor: **Kiyohara, Takao**
**14-7, Mefu 2-chome**
**Takarazuka-shi Hyogo(JP)**

(72) Inventor: **Fukui, Masaru**
**10-1-141, Sonehigashimachi 2-chome**
**Toyonaka-shi Osaka-fu(JP)**

(72) Inventor: **Fukui, Akio**
**3-27, Sakuramachi**
**Nishinomiya-shi Hyogo(JP)**

(72) Inventor: **Ogino, Shigeo**
**25-28-309, Hinoikecho**
**Nishinomiya-shi Hyogo(JP)**

(72) Inventor: **Inaba, Makoto**
**979-6, Tsukuicho-Nakano**
**Tsukui-gun Kanagawa(JP)**

(72) Inventor: **Tsukagoshi, Shigeru**
**6-13-17, Shimoshakujii**
**Nerima-ku Tokyo(JP)**

(72) Inventor: **Sakurai, Yoshio**
**2-24-13, Shimorenjaku**
**Mitaka-shi Tokyo(JP)**

(74) Representative: **Myerscough, Philip Boyd et al,**
**J.A.Kemp & Co. 14, South Square Gray's Inn**
**London, WC1R 5EU(GB)**

(54) **Antitumor substance and composition, and process for preparing said substance.**

(57) The toxicity of a known antitumor substance prepared by culturing a microorganism, *Acinetobacter* calcoaceticus var. microformis SC-1714 is reduced by treatment with barium hydroxide and isolating an antitumor substance having an excellent antitumor activity as well as a potent immunostimulatory activity. The novel substance is effective when administered in very small amounts without harmful effects such as toxicity.

Croydon Printing Company Ltd.

DESCRIPTION                          **0024206**

**"ANTITUMOR SUBSTANCE AND COMPOSITION, AND
PROCESS FOR PREPARING SAID SUBSTANCE"**

The present invention relates to antitumor substance having an excellent antitumor activity as well as a potent immunostimulatory activity, and to a process for producing the same, which comprises culturing a microorganism belonging to the genus Acinetobacter in a suitable culture medium and thereafter isolating antitumor substance therefrom.

It has been known that the microorganism named as Acinetobacter calcoaceticus var. microformis SC-1714, which is deposited at American Type Culture Collection, U.S.A. under the deposition No. ATCC-31299 as well as at Fermentation Research Institute, Agency of Industrial Science and Technology, Japan, under the deposition No. FERM-P-4064, produces an antitumor substance [German Patent Publication (Offenlegungsschrift) No. 2829975; U.S. Patent No. 4,209,507].

As the result of a study on the production of said antitumor substance, it has been found that an improved antitumor substance having a very low toxicity can be obtained from said known antitumor substance by treating it with barium hydroxide.

The antitumor substance of the present invention has been found to be very effective in inhibiting growth of experimental tumor cells as well as in extinguishing solid tumors. In addition thereto, the antitumor substance of the present invention shows a potent immunostimulatory activity at a very low dose. Thus, the antitumor substance of the present invention not only has a direct growth-inhibitory effect on tumor cells but also increases immunity of a tumor-bearing host. Moreover, the antitumor substance of the present invention has a very low toxicity.

Thus, the present invention provides antitumor and immunostimulative substance useful for the treatment of tumors and a pharmaceutical composition thereof, and it also provides a process for producing the same.

The antitumor substance of the present invention can be prepared by treating the substance produced by said microorganism, Acinetobacter calcoaceticus var. microformis SC-1714 (hereinafter referred to as "Substance SC-1714) with barium hydroxide.

The Substance SC-1714 used as the starting material of the production of the antitumor substance is already known and can be prepared by the method disclosed in said German Patent Publication (Offenlegungsschrift) No. 2829975, and U.S. Patent No. 4,209,507.

The Substance SC-1714 can be prepared by culturing Acinetobacter calcoaceticus var. microformis SC-1714, in a suitable medium and thereafter isolating it from the culture broth.

In the following, the characteristics of Acineto-bacter calcoaceticus var. microformis SC-1714 are described:

1. Morphology

Cells spherical, sometimes short rod-shaped, occuring singly, in pairs or aggregates. Dimensions, 0.6 to 1.1 microns in diameter. Non-motile. No spores formed. Gram-negative. Acid-fast stain negative.

2. Growth on various culture media

(1) Agar-bouillon plate (cultivated at 28°C for 48 hours)

Abundant growth with rising. Mucoid colonies with peripheral completely circular to wavy, white to pale reddish yellow in color, with dull lustre and intransparent.

(2) Agar-bouillon slant (cultivated at 28°C for 48 hours)

Abundant growth with rising. Colonies smooth and mucoid, white to pale reddish yellow in color, with dull lustre and intransparent.

      (3) Liquid bouillon (cultivated at 28°C for 24 hours)

           Moderately turbid, with formation of fragile pellicles and viscous sediments.

      (4) Bouillon-gelatin stab (cultivated at 28°C for 30 days)

           Growth on surface. Colonies mucoid and not liquefied.

      (5) Litmus milk (cultivated at 28°C for 4 days)

           Acidified with coagulation.

      (6) Media King A and King B (cultivated at 28°C for 7 days)

           Pale yellow diffusible pigments formed in both media.

3. Physiological properties:

      (1) Growth temperature: 20° to 45°C, optimum temperature being 30° to 37°C

      (2) pH range: 5.0 to 8.5, optimum pH range being about 7.0

      (3) Nitrates: not reduced

      (4) Denitrification reaction: negative

      (5) MR test: negative

      (6) VP test: negative

      (7) Indole formation: none

      (8) Sulfide formation: none

      (9) Starch hydrolyzed

   (10) Citric acid utilized

   (11) Utilization of nitrogen sources: Growth possible by utilizing ammonium salts

   (12) Urease: absent

   (13) Catalase: present

   (14) Oxidase: absent

   (15) Aerobic

   (16) O-F test: oxidative

   (17) Acid and gas formation from carbohydrates:

           Acid is formed but no gas formed from xylose, glucose, mannose, galactose, lactose, and dextrin; neither acid nor gas is formed from arabinose, fructose, maltose, saccharose, trehalose, raffinose, sorbitol, innositol, mannitol, glycerine, starch, inulin, salicin, α-methyl glucoside.

(18) Cellose decomposed

(19) Lysine decarboxylase activity: weakly positive

(20) Haemolysis: positive

(21) Susceptibility to antibiotics: resistant to 20 units of penicillin G

(22) Nutrient demands: No special demand, with sufficient growth in a simple medium such as peptone water

The above characteristics are substantially the same as those of genus Acinetobacter, (The genus Acinetobacter has only one species, namely Acinetobacter calcoaceticus) belonging to the family of Neisseriaceae, as described in Bergey's Manual of Determinative Bacteriology, eighth edition, 1974. But, when compared in further detail, Acinetobacter calcoaceticus is shaped primarily in short rods, sometimes in spheres, with dimensions of 1.0 to 1.5 by 1.5 to 2.5 microns, as different in shape and dimensions from the present strain. Accordingly, the strain is identified to be a variant of Acinetobacter calcoaceticus and named as Acinetobacter calcoaceticus var. microformis SC-1714 and deposited at American Type Culture Collection, U.S.A., under the deposition No. ATCC-31299 and also at Fermentation Research Institute, Agency of Industrial Science and Technology, Japan, under the deposition No. FERM-P-4064.

In order to obtain the Substance SC-1714, said microorganism is inoculated onto a suitable agar medium and cultivated at a suitable temperature, preferably in the range from 25° to 37°C, most preferably at 30°C, for several days, preferably from 2 to 3 days. This seed culture, after being confirmed of absence of contamination, is then inoculated in a liquid or a solid medium to carry out cultivation at a suitable temperature.

The cultivation can be carried out under a
stationary way, shaking, aeration or aeration and stirring.
The solid medium includes agar, gelatin, starch, etc. or a
suitable combination thereof.  As a cultivation for producing
the Substance SC-1714, either liquid or solid medium can be
used.  But a liquid medium is more convenient from standpoint
of handling and productivity.  Conventional media can sufficiently be used as a medium for cultivation of the microorganism producing the substances, so far as nutrient
ingredients necessary for growth of the above organism and
production of said substance are contained in the medium.
Namely, glucose, maltose, lactose or sugar molasses can be
used as carbon source; peptone, meat extract, malt extract,
corn steep liquor, yeast extract, distillers, soybean
proteolysis products, or amino acids as nitrogen source.
If desired, inorganic nitrogen source such as ammonium salts
can also be used.  Other inorganic salts, including phosphoric
acid salts, magnesium salts, sodium salts, iron salts, or
various vitamins can also optionally be added.

The initial pH for cultivation is adjusted at about
neutral values, preferable at 6.0 to 7.5.  The cultivation
temperature is variable within the range at which said
microorganism can grow and produce said substance, but it
preferably falls within the range from 25° to 35°C.  The
cultivation time differs depending on the cultural conditions, but usually from one to two days.  Cultivation may be
terminated optimally at the time when the amount of said
substance accumulated in the medium appears to be at its
maximum.  As mentioned above, either stationary, shaking,
aerating or aeration-stirring cultivation may be available.
Among them, shaking or aeration-stirring cultivation is
preferred.  Shaking culture may suitable be carried out at
a stroke of 5 to 20 cm at the rate of 30 to 200 r.p.m.;
while aeration-stirring cultivation under aeration of 0.1 to 2.0 liter/liter-medium/min. at a stirring speed of 30 to 800 r.p.m.

After completion of the cultivation, the microorganism cells are removed from the cultivation medium.

Removal of microbial cells can be conducted, after adjusting pH of the culture broth, if necessary, by way of centrifugation or filtration using filtrating aids such as hyflo-super cells. Centrifugation method is better in efficiency of separation. For the purpose of avoiding re-growth of said microorganism which may possibly be remained in the culture broth even after removal of microbial cells by means of centrifugation, etc., the filtrate is further subjected to removal of cells using a bacteria filter, such as Millipore filter HAWPO 2500 (0.45 micron or less; trade name, Millipore Co., U.S.A.) or _Seitz_ bacteria filter. Furthermore, if necessary, an antibiotic substance such as streptomycin may be added at a final concentration of, for example, 100 mcg/ml or more prevention of growth of said microorganism or other bacteria floating in the air which may possibly be contaminated during operational procedures. The thus treated filtrate, with or without addition of said antibiotic substance, can be stored by being filled in a sterilized ampoule, followed by sealing under aseptic conditions.

After the removal of the microorganism cells, a hydrophilic organic solvent such as acetone, methanol or ethanol is added to the culture broth under cooling to precipitate crude substance SC-1714. In precipitating the substance SC-1714, a water-soluble organic solvent such as acetone, methanol, ethanol or a mixture thereof is added to the culture broth in a large amount under cooling, preferably at around 5°C and the mixture is allowed to stand for about one night at a low temperature preferably at 5°C. In order to precipitate the substance, it is necessary to add the solvent in such an amount that the concentration of the solvent in the culture broth is no less than 60 %. The precipitation of the substance may be accelerated by adding powders of calcium chloride in an amount of 0.1 to 1 % (W/V) of the culture broth, preferably 0.8 % (W/V) before the addition of the solvent. The resulting precipitates are collected by filtration or decantation.

If desired, the crude substance SC-1714 obtained as above is purified by dissolving it into water and precipitating it in the same manner as above.

Alternatively, the crude substance SC-1714 may be obtained in a form of a condensed solution by concentrating the culture broth after the removal of the microorganism cells. The concentration of the culture broth can be carried out by passing it through an ultrafiltration membrane, whereby the salts, water and low molecular weight substances contained therein are removed and the culture broth is condensed to 1/10 to 1/20 of its original volume. As a method for collecting the substance SC-1714, this condensation of the culture broth with an ultrafiltration membrane is advantageous over the precipitation with an organic solvent in term of yield of the substance.

As an ultrafiltration membrane, either flat type or hollow fiber type can be used.

For the purpose of a further purification, the precipitates or the condensate obtained as above are usually dissolved in water and then deionized and decolored with ion-exchange resins such as Dowex 1x2, Dowex 50, Duolite S-30, and the like. Prior to said deionization and decoloration, salting out may be carried out by saturating the solution with an inorganic salt such as ammonium sulfate. Purification treatments can be carried out in various orders, for example, in the order of salting out, dialysis and deionization or salting out, deionization and dialysis, etc. A part of these treatments can also be used in combination, if desired. Then, a water-soluble organic solvent such as acetone, methanol, or ethanol is added to the solution obtained as above to precipitate the substance SC-1714, which is then washed with the same solvent (concentration: 90 % or more) and dried with acetone or ether, or dissolved to water and lyophilized.

The substance SC-1714 obtained as above is then dissolved in water, if necessary and them subjected to a defatting operation according to the method of Folch et al [J. Folch et al, The Journal of Biological Chemistry, Vol. 226, p.497, (1957)], wherein the aqueous solution is treated with a mixed solvent of chloroform and methanol and the aqueous layer is then either lyophilized or evaporated at a low temperature (e.g. 35°C - 40°C) under a reduced pressure. By this treatment, certain substance is eliminated from the substance to increase the solubility in water of the substance.

The substance SC-1714 defatted as above is then subjected to ion-exchange chromatography with a cation-exchanger such as carboxymethyl-Sephadex (CM-Sephadex). The non-adsorbed fractions eluted by water are adsorbed an anion-exchanger such as diethylaminoethyl cellulose, which is then washed with water and eluted with an aqueous solution of salts, preferably 0.5 - 1.0 M NaCℓ aqueous solution. After collecting the fractions having an antitumor activity, the eluates was subjected to desalting operation with the ultrafiltration module ACL-1010 (Asahi Chemical Industry Limited, Japan) and then lyophilized to give the substance SC-1714.

In the present invention, the antitumor substance of the present invention can be prepared by treating the substance SC-1714 with barium hydroxide, neutralizing with an acid (e.g. 1N - 3N hydrochloric acid) and then removing the salts formed in the previous operation in a conventional manner, preferably with an ultrafiltration.

The amount of barium hydroxide to be added to is not particularly limited, but 2 - 15 parts by weight, preferably 4 - 10 parts by weight of barium hydroxide is usually added to 1 part by weight of the lyophilized substance SC-1714.

For example, the substance SC-1714 is dissolved in a small amount of water and then added to a mixture of barium hydroxide and water (barium hydroxide : water = 1 : 25 - 60 by weight). After stirring for one hour, the unsolubles are centrifugally removed from the mixture at 5°C. The resulting supernatant is neutralized with 1 - 3N hydrochloric acid, deionized with a conventional procedures such as dialysis with cellophane tube, ultrafiltration, molecular sieve chromatography, etc., and lyophilized to give the objective antitumor substance of the present invention.

The antitumor substance of the present invention is a non-hygroscopic powder showing no distinct melting point, and an aqueous solution thereof is substantially neutral or weakly acidic. The substance of the present invention is found to contain 15 - 24 % of protein calculated as bovine serum alubumin by quantitative analysis of protein by Copper-Folin method, 28 - 54 % of saccharide calculated as glucose by quantitative analysis of, neutral saccharide by phenol-sulfuric acid method, and 0.9 - 12 % of fatty acid by the gas chromatographic analysis.

These values are not always constant because the compositions of the product obtained will vary depending on the culture conditions and the method of purification.

The antitumor substance of the present invention is weakly positive to ninhydrin reaction. Its antitumor activity is weakened when decomposed with a proteolytic enzyme such as pronase, papain, etc. When this substance is partially decomposed with a carbohydrase such as glycosidase, its antitumor activity is also weakened. On the other hand, when this substance is decomposed at a high temperature in a strongly acidic or alkaline solution, its biological activities such as antitumor activity are completely vanished.

The physical, chemical and biological properties of the antitumor substance of the present invention are as follows:

Physical and chemical properties

1. Appearance and solubility: obtained as white powders or films, soluble in water but insoluble in organic solvents

2. Decomposition point: higher than 235°C

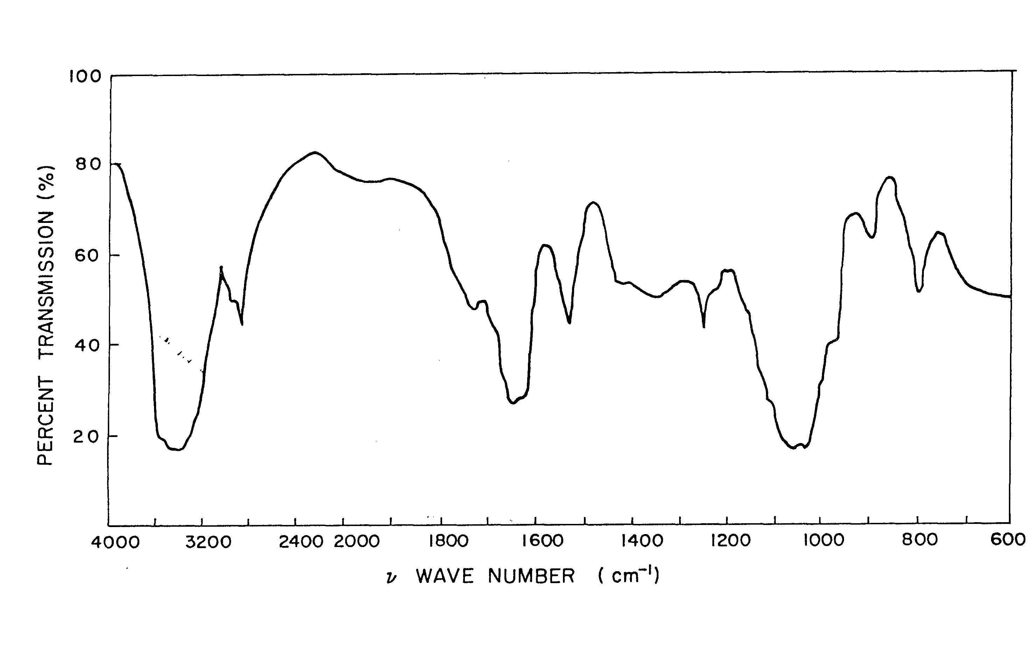

3. Infrared absorption spectrum: (KBr-Tablet) as shown in the Drawing, specific absorptions, 3400, 2900, 1650, 1540 and 1100 - 1000 cm$^{-1}$

4. Elemental analysis:
C 32.2 - 41.9 %, H 4.5 - 7.2 %,
N 2.3 - 5.8 %

5. Color reactions: slightly positive or positive to ninhydrin reaction, xanthoprotein reaction and biuret reaction; positive to Molisch reaction, anthrone-sulfuric acid reaction, phenolsulfuric acid reaction and carbazole-sulfuric acid reaction

6. Color reaction of hydrolyzed products: ninhydrin reaction intensified (after hydrolysis in 1N HCℓ, at 105°C for 24 hours); negative to phloroglucin reaction (after hydrolysis in 1N H$_2$SO$_4$ at 100°C for 4 hours)

7. Stability: Stable at pH 5 to 8 at 0° to 60°C

Biological properties

       1.  Biological activity: having no antimicrobial activity, having antitumor activity, immunostimulating activity, anti-vaccinia virus activity, anti-inflammatory activity and activity for inhibiting secretion of acid in the stomach

       2.  Acute toxicity: acute toxicity for mouse (ICR type, male, 20 to 25 g) by a single dosage is as follows;

$$LD_{100}\ 300\ mg/kg\ (intraperitoneal\ and\ intravenous\ injection)$$

The antitumor substance of the present invention are useful for the treatment of tumors in a warm-blood animal, and for this purpose the substance is orally or parenterally administered at a daily dosage of 0.1 - 10 mg/Kg.

The following example is given to illustrate the present invention more precisely, but the present invention is not limited thereto.

Example 1

In a 100 ℓ vessel was placed 60 ℓ of a culture medium (pH 7.0) containing 1.0 % glucose, 1.0 % peptone, 1.0 % meat extract and 0.5 % sodium chloride, and the medium was sterilized at 120°C for 20 minutes. After the medium cooled, 0.6 ℓ of culture broth wherein Acinetobacter calcoaceticus var. microformis SC-1714 (ATCC-31299: FERM-P-4064) was previously cultured in a medium having the same composition as above was added to the culture medium and cultured at 30°C for 20 hours. After the culture broth was neutralized, the microorganism cells were centrifugally removed therefrom at 10,000 r.p.m., while cooling to 5°C.

To 30 ℓ of the culture broth was gradually added 240 g of anhydrous calcium chloride with stirring. Thereafter, 45 ℓ of acetone was added to the culture broth at 5°C.

The resulting precipitates were dissolved in 5 ℓ of water. The solution was passed through a column equipped with 1.5 ℓ of Dowex 1x2 (Cℓ Type) and then lyophilized to give 40 g of crude powder.

30 ℓ of the culture broth from which the microorganism cells were removed was passed through an ultrafilter module ACL-1010 (made by Asahi Chemical Industry Company Limited, Japan, a hollow fiber type polyacrylonitrile membrane capable of separating substances of 13,000 or less of molecular weight is used), whereby the culture broth was condensed to 3 ℓ.

Thus condensed culture broth was passed through a column equipped with Dowex 1x2 (Cℓ Type) and then lyophilized to give 47 g of crude powder, which was found to be as effective as the crude powder obtained as above with regard to antitumor activity against Sarcoma 180. Thus prepared crude powders were combined, 80 g of which was mixed with 200 mℓ of water and added to 6 ℓ of a mixed solvent of chloroform (4 ℓ) and methanol (2 ℓ). The mixture was vigorously stirred and the precipitates were collected by filtration and decantation. The precipitates were again mixed with 200 mℓ of water and added to a mixed solvent of 4 ℓ of chloroform and 2 ℓ of methanol.

After the mixture was vigorously stirred for 2 hours, the precipitates were freeze-dried in a conventional manner to give 5 g of white powders. The powders were dissolved in a small amount of water and filled in a column (60 mm in diameter : 600 mm in length) equipped with 1.5 ℓ of CM-Sephadex. After eluting with water, the fractions having an antitumor activity are combined and filled in a column (60 mm in diameter; 500 mm in length) equipped with 1 ℓ of diethylaminoethyl cellulose. After washing with water, the active substance, the substance SC-1714 was eluted with 1 ℓ of 1 M sodium chloride aqueous solution. The eluate was desalted with the ultrafiltration membrane and condensed to 50 mℓ, which was then added to a mixture of 6 g of barium hydroxide and 250 mℓ of water and stirred for one hour at a room temperature. The precipitates were removed with a centrifuge at 5°C at 9000 r.p.m. for 20 minutes. The resulting supernatant was neutralized with 3N hydrochloric acid, desalted with the ultrafiltration membrane and lyophilized to give 1.2 g of the objective antitumor substance as white powders.

Elemental analysis: C 41.8 %, H 7.2 %, N 4.1 %

Protein content: 22.4 %

Saccharide content: 51.8 %

fatty acid content: 1.2 %

Thus obtained antitumor substance of the present invention has 120 mg/Kg of $LD_{50}$ value, while the substance SC-1714, from which the antitumor substance of the present invention was derived, has 60 mg/Kg of $LD_{50}$ value when tested using ICR-mice.

The antitumor activities of the compound of the present invention against Lewis lung carcinoma, Melanoma B-16, Colon 26 and Colon 38 adenocarcinomas, Sarcoma 180, Ehrlich carcinoma were estimated and the results were shown in the following Table 1.

Table 1

| Transplantation of tumors | | Drug administration | | Life prolongation (T/C) * | | | | | | |
|---|---|---|---|---|---|---|---|---|---|---|
| | | | | Dose (mg/kg/day) | | | | | | |
| Tumors | Method | Route | Schedule | 20 mg/kg | 10 mg/kg | 5 mg/kg | 2.5 mg/kg | 1.25 mg/kg | 0.62 mg/kg | 0.31 mg/kg |
| Lewis lung carcinoma | Sub-cutaneous injection | Intravenous injection / Intra-peritonealy | once a day for 9 days after the transplantation | | 148 % | 145 % | 145 % | 150 % | 154 % | |
| | | | once a day for 9 days after the transplantation | | 153 % | 137 % | 132 % | 145 % | 161 % | 132 % |
| | | | once a day on Days 1, 5 and 9 after the transplantation | 178 % | 153 % | 148 % | 156 % | 147 %. | | |
| | | Intravenously | once a day on Days 1, 5 and 9 after the transplantation | | 145 % | | 120 % | | 149 % | |
| | | orally | once a day on Days 1, 3, 5, 7 and 9 after the transplantation | | 149 % | 141 % | | | | |
| | | | once a day on Days 1, 3, 5,7,9,11,13 and 15 after the transplantation | | | 189 % | | | | |
| Melanoma B-16 | Intra-peritoneal injection | Intra-peritoneally | once a day for 9 days after the transplantation | | 119 % | 110 % | 110 % | 123 % | 130 % | |
| Colon 26 | | | once a day on Days 1 and 5 after the transplantation | 154 % | 193 % | 162 % | | | | |
| Sarcoma 180 | | | once a day for 9 days after the transplantation | | 123 % | 174 % | 150 % | 131 % | 143 % | 120 % |
| Ehrlich carcinoma | | | once a day on Days 1, 5 and 9 after the transplantation | | 152 % | 243 % | 223 % | 123 % | 115 % | |

* T/C % = (Average of survival days for treated group of mice/average of survival days for Control group of mice) x 100

The inhibitory effect of the antitumor substance of the present invention on the growth of solid tumor of Colon 38 was observed.

Colon 38 was transplanted to mice by an subcutaneous injection on Day 0.

The antitumor substance of the present invention was intraperritonealy administered to the mice once a day on Days 2 and 9 after the transplantation.

On Day 20, solid tumors were taken out and compared with those of control group. The results were shown in the following Table 2.

### Table 2

Inhibition of tumor mass growth of Colon 38 adenocarcinoma

| Dosage | Inhibitory percentage |
| --- | --- |
| 10 mg/Kg/day | 71 % |
| 5 mg/Kg/day | 50 % |
| 2.5 mg/Kg/day | 49 % |

The antitumor substance of the present invention was found to increase immunity of mouse in the experiment wherein 2 mg/Kg of the substance was subcutaneously administered to mice previously sensitized with sheep red blood cells once a day for 4 days, and the number of plaque forming cells (PFC) in the spleen was measured. PFC number of treated mice was increased three times as much as that of control group.

CLAIMS

1. A substance having an antitumor activity and an immunostimulating activity which is prepared by

(1) culturing the microorganism named as <u>Acinetobacter</u> <u>calcoaceticus</u> <u>var.</u> <u>microformis</u> <u>SC-1714</u> in a suitable medium,

(2) isolating substance having an antitumor activity,

(3) treating the substance with barium hydroxide, and

(4) isolating the objective substance therefrom.

2. A substance having an antitumor activity and an immunostimulating activity according to Claim 1 which is prepared by culturing said microorganism in a suitable media, isolating substance having an antitumor activity, treating the substance with barium hydroxide and then isolating therefrom the substance having the following properties:

<u>Physical and chemical properties</u>

1. Appearance and solubility: obtained as white powders or films, soluble in water but insoluble in organic solvents

2. Decomposition point: higher than 235°C

3. Infrared absorption spectrum: (KBr-Tablet) as shown in the Drawing, specific absorptions, 3400, 2900, 1650, 1540 and 1100 – 1000 $cm^{-1}$

4. Elemental analysis:
   C 32.2 – 41.9 %, H 4.5 – 7.2 %
   N 2.3 – 5.8 %

5. Color reactions: slightly positive or positive to ninhydrin reaction, xanthoprotein reaction and biuret reaction; positive to Molisch reaction, anthrone-sulfuric acid reaction, phenol-sulfuric acid reaction and carbazole-sulfuric acid reaction

6. Color reaction of hydrolyzed products: ninhydrin reaction intensified (after

- 17 -

hydrolysis in 1N HC$\ell$, at 105°C for 24 hours); negative to phloroglucin reaction (after hydrolysis in 1N $H_2SO_4$ at 100°C for 4 hours)

7. Stability: Stable at pH 5 to 8 at 0° to 60°C

Biological properties
_____

1. Biological activity: having no anti-microbial activity, having antitumor activity, immunostimulating activity, anti-vaccinia virus activity, anti-inflammatory activity and activity for inhibiting secretion of acid in the stomach

2. Acute toxicity: acute toxicity for mouse (ICR type, male, 20 to 25 g) by one dosage is as follows:

$LD_{100}$ 300 mg/kg (intraperitoneal and intravenous injection)

3. An antitumor composition which comprises as an active ingredient a pharmaceutically effective amount of a substance as claimed in claim 1 or 2 and a pharmaceutically acceptable carrier.

4. A substance as claimed in claim 1 or 2 for use as an antitumor agent.

5. A process for producing a substance having an antitumor activity and an immunostimulating activity which comprises culturing the microorganism named as <u>Acinetobacter calcoaceticus var. microformis SC-1714</u> in a suitable medium and isolating a substance having an antitumor activity, characterised by treating the substance with barium hydroxide and isolating the substance having an antitumor activity and an immuno-stimulating activity.

0024206